(19) Europäisches Patentamt

European Patent Office

Office européen des brevets

(11) **EP 4 001 291 A1**

(12) **EUROPEAN PATENT APPLICATION**
published in accordance with Art. 153(4) EPC

(43) Date of publication:
**25.05.2022 Bulletin 2022/21**

(21) Application number: **20839773.7**

(22) Date of filing: **07.07.2020**

(51) International Patent Classification (IPC):
**C07K 1/18** (2006.01)      **C07K 1/20** (2006.01)
**C07K 1/22** (2006.01)      **C07K 14/565** (2006.01)

(52) Cooperative Patent Classification (CPC):
**C07K 1/18; C07K 1/20; C07K 1/22; C07K 14/565**

(86) International application number:
**PCT/KR2020/008847**

(87) International publication number:
**WO 2021/010638 (21.01.2021 Gazette 2021/03)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **18.07.2019 KR 20190087244**

(71) Applicant: **Abion Inc.**
**Seoul 08394 (KR)**

(72) Inventors:
• **CHOI, Jun Young**
  **Seoul 08394 (KR)**
• **KIM, Na Young**
  **Seoul 08394 (KR)**
• **HONG, Sung Hyun**
  **Seoul 08394 (KR)**

(74) Representative: **Schwarz & Partner Patentanwälte GmbH**
**Patentanwälte**
**Wipplingerstraße 30**
**1010 Wien (AT)**

(54) **METHOD FOR PURIFYING DIGLYCOSYLATED INTERFERON-BETA PROTEIN**

(57) The present invention relates to a method for purifying a diglycosylated interferon-beta protein and, more specifically, to a method for purifying a diglycosylated interferon-beta protein, wherein a culture solution containing interferon-beta expressed in host cells is obtained, and subjected to affinity chromatography, low-pH inactivation, hydrophobic interaction chromatography, anion-exchange chromatography, and cation-exchange chromatography, and to a diglycosylated interferon-beta protein separated by the method.

FIG. 1

EP 4 001 291 A1

**Description**

**TECHNICAL FIELD**

This application claims the priority of Korean Patent Application No. 10-2019-0087244, filed on July 18, 2019, the entirety of which is a reference of the present application.

The present invention relates to a method for purifying a diglycosylated interferon-beta protein, and more particularly, to a method for purifying a diglycosylated interferon-beta protein and a diglycosylated interferon-beta protein separated by the method, wherein the method includes steps of (a) obtaining a culture solution comprising interferon-beta expressed in a host cell; (b) performing affinity chromatography for the culture solution obtained in step (a); (c) low pH inactivating the solution obtained in step (b); (d) performing hydrophobic interaction chromatography for the solution obtained in step (c); (e) performing anion-exchange chromatography for the solution obtained in step (d); and (f) performing cation-exchange chromatography for the solution obtained in step (e).

**BACKGROUND ART**

Human interferon (IFN) is currently known as 8 types, which are classified as two types of Type 1 and Type 2 according to physicochemical and functional features. The Type 1 interferon includes alpha-, beta-, kappa-, delta-, epsilon-, tau- and omega-interferons and the Type 2 interferon includes gamma-interferon.

The interferons (IFNs) suppress the replication of virus to exhibit antiviral activity, suppress cell proliferation, and regulate the immune response. Among them, the interferon-beta is a cytokine produced in a reaction by viral infection and the like, and particularly, is used as a multiple sclerosis (MS) therapeutic agent and the like, and also exhibits the anticancer activity to be used as a cancer therapeutic agent.

Currently, there are two types of interferon-beta to be used for treatment. First, interferon-beta-la is a glycosylated protein which is produced from the chinese hamster ovary (CHO) including a human interferon-beta gene, consists of 166 amino acid residues, and has a size of 25 kD. Second, interferon-beta-lb is a protein consisting of 165 amino acid residues produced from E. coil, wherein a sugar is deleted, a methionine residue as an amino acid at position 1 is deleted, and a cysteine residue at position 17 is substituted with serine. Interferon-beta-la which has been currently commercialized includes Rebif and Avonex, and interferon-beta-lb includes Betaseron, and Extavia.

On the other hand, a glycoprotein is a protein containing an oligosaccharide chain that is covalently attached to a polypeptide side chain. The glycoprotein has very various physiological functions, including structure, protection, carrying, hormone or enzyme functions, and it is generally known that the attached sugar chain importantly acts to these physiological functions. The glycoprotein may be currently produced in recombination, but requires a wide purification procedure to extract a targeted glycoprotein from a cell culture collection. Since the detachment, modification, and the like of the sugar chain during production/expression and purification processes affect the physiological functions, it is important to configure each process so as to be formed correctly in the production/expression process or so as not to detach and modify the sugar chain in the purification process.

The interferon is also a kind of glycoprotein, and in the case of wild-type interferon-beta, the sugar chain is attached to an amino acid at position 80. Particularly, in 2004, an interferon-beta mutant protein causing mutation to glycosylation of interferon has been developed, and studies to use the protein as a therapeutic agent have been conducted. In order to use the interferon-beta as a therapeutic agent, the interferon-beta has a sufficient protein amount and high purity and should be purified in a form suitable for long-term preservation. Since the interferon-beta is less stable, in the purification process, decomposition reactions, such as cleavage of a peptide bond, deamidation, and oxidation of methionine to methionine sulfide, and disulfide exchange, commonly occur (US2012/0177603), but it is necessary to consider these decomposition reactions in the purification process.

In addition, in a general purification process, since a plurality of different chromatography methods can be used, in order to find a suitable purification method, a large number of combinations needs to be tested. In these combinations, different orders, even different numbers of chromatography methods may be combined and used, but according to these combinations, a purification effect is not sufficient or the purified protein may be damaged, and as a result, in order to purify the glycosylated protein, a method of determining chromatography steps in a suitable order is always required in the art.

In particular, in the related arts, there are disadvantages that since organic solvents are used in the purification process, special equipment is required in large-scale production, and since the purification process is complicated, high cost is required. Therefore, it is necessary to develop a method for purifying a diglycosylated interferon-beta protein which is easy in a large amount of purification, simple and economical, and has high yield without using the organic solvents.

**DISCLOSURE**

**TECHNICAL PROBLEM**

Therefore, the present inventors have conducted a study to develop a method for purifying a diglycosylated interferon-beta protein, and as a result, found that purification using affinity chromatography, hydrophobic interaction chromatography, and ion exchange chromatography was a new purification method which had a sufficient protein amount and high purity, and had less modification of glycosylation or other modifications of interferon-beta, and then completed the present invention.

Therefore, an object of the present invention is to provide a method for purifying a diglycosylated interferon-beta protein including steps of (a) obtaining a culture solution comprising interferon-beta expressed in a host cell; (b) performing affinity chromatography for the culture solution obtained in step (a); (c) low pH inactivating the solution obtained in step (b); (d) performing hydrophobic interaction chromatography for the solution obtained in step (c); (e) performing anion-exchange chromatography for the solution obtained in step (d); and (f) performing cation-exchange chromatography for the solution obtained in step (e).

Another object of the present invention is to provide a diglycosylated interferon-beta protein obtained by the purification method of the present invention.

**TECHNICAL SOLUTION**

In order to achieve the object, the present invention provides a method for purifying a diglycosylated interferon-beta protein including steps of (a) obtaining a culture solution comprising interferon-beta expressed in a host cell; (b) performing affinity chromatography for the culture solution obtained in step (a); (c) low pH inactivating the solution obtained in step (b); (d) performing hydrophobic interaction chromatography for the solution obtained in step (c); (e) performing anion-exchange chromatography for the solution obtained in step (d); and (f) performing cation-exchange chromatography for the solution obtained in step (e).

In order to achieve another object, the present invention provides a diglycosylated interferon-beta protein obtained by the purification method of the present invention.

Hereinafter, the present invention will be described in detail.

General chromatography methods and applications thereof are known to those skilled in the art.

The term "chromatography material" used herein means a solid material that may be used without additional modification as a chromatography material, for example, hydroxyapatite or an affinity chromatography material, and means a material including a bulk core material to which a chromatography functional group is preferably attached by a covalent bond. The bulk core material represents that a chromatography process, that is, interaction between polypeptide to be separated and a chromatography functional group of the chromatography material is not substantially performed. The chromatography material serves to provide a 3D skeleton secured so that a solution containing a material to be simply attached with or detached from the chromatography material may approach the chromatography functional group. Preferably, the bulk core material is a solid form. Accordingly, preferably, the "chromatography material" is a solid phase to which the chromatography functional group is preferably attached by a covalent bond. Preferably, the "chromatography functional group" is an ionizable hydrophobic or hydrophobic group, a composite group in which different chromatography functional groups are combined to bind to only some types of polypeptides, or a covalent-bonded charged group.

The term "polypeptide" and "protein" used herein are used according to a conventional meaning, that is, a polymer of amino acid residues. The polypeptide is not limited to a specific length, but in the context of the present invention, may generally refer to a fragment of a full length protein. The polypeptide or protein may include modification after translation, for example, glycosylation, acetylation, phosphorylation, etc., and other modifications (naturally occurring modifications and non-naturally occurring modifications) known in the art. The polypeptide and protein of the present invention may be prepared using any of various known recombinant and/or synthetic techniques.

The terms "bind mode" and "elute mode" used herein mean operation types of a chromatography method in which a solution containing a desired material (e.g., diglycosylated interferon-beta) is in contact with a static phase, preferably a solid phase, wherein the desired material binds to a solid-phase chromatography material. As a result, the desired material is attached to a solid phase, while an undesired material is removed along a flow of a solvent or together with a supernatant. Thereafter, the desired material is eluted from the solid phase and recovered from the solid phase together with an elution solution. It is not necessarily meant that the undesired material is fully removed, but 50% or more, preferably 75% or more, more preferably 85% or more, most preferably 95% or more of the undesired material is removed.

The terms "continuous elution" and "continuous elution method" used herein mean, for example, that the concentration of a material causing elution, that is, separation from the chromatography material of the binding material is continuously increased or decreased. That is, the concentration is changed by a series of small steps of changing the concentration of the material causing the elution to be not greater than 2%, preferably 1% in each step. In this "continuous elution"; one or more conditions of the chromatographic method, for example, pH, ionic strength, a concentration of salt, and/or a flow rate are linearly changed or geometrically changed. Preferably, the change is linearly changed.

The terms "stepwise elution" and "stepwise elution method" used herein mean, for example, a chromatography method in which the concentration of the material causing elution, that is, separation from the chromatography material of the binding material is continuously increased or decreased once, i.e., at a value/level to a next value/level. In the "stepwise elution", one or more conditions of the chromatographic method, for example, pH, ionic strength, a concentration of salt, and/or a flow rate are changed once from a first value, for example, a start value to a second value, for example, a final value. In the change in the step, the concentration of the material causing the elution is changed to be greater than 5%, preferably 10%. The "stepwise elution" is changed incrementally, that is, stepwisely in contrast to the linear change. In the "stepwise elution method", a new fraction is collected after each increase. After each increase, the condition is maintained to the next step in the elution method.

In the present specification, the terms "separated protein" and "separated polypeptide" are polypeptides which are substantially no contaminated cell component such as other protein impurities related to hydrocarbons, lipids or polypeptides in nature. Typically, the preparation of the separated protein contains very purified, i.e., about 80% pure, about 90% or more pure, about 95% or more pure, 95% or more pure, or 99% or more pure polypeptides. A method of showing that a specific protein formulation contains separated polypeptides is to show a single band, for example, after electrophoresing the protein preparation and dyeing a gel with Coomassie Brilliant Blue. However, the term "separated" does not exclude the existence of the same polypeptide in a different physical form, such as a dimer, a derivatized form, a correctly unfolded form, an incorrectly disulfide crosslinked form or scrambled form.

The term 'polynucleotide' or 'nucleic acid' used herein refers to deoxyribonucleotide (DNA) or ribonucleotide (RNA) in a single- or double-stranded form. Unless otherwise limited, the 'polynucleotide' or 'nucleic acid' also includes known analogs of natural nucleotides that hybridize to nucleic acids in a similar manner to those of naturally generated nucleotides.

The term "activity of human interferon-beta" used herein is defined as one or more activities that any polypeptide is sufficient to be identified as human interferon-beta among the known activities of human interferon-beta. These activities may include, for example, reduction, alleviation or treatment activity for multiple sclerosis, antivirus activity, cell growth inhibition activity, anti-growth activity, anti-proliferative activity, lymphocyte cell toxicity enhancement activity, immune regulatory activity, differentiation induction or inhibition activity of target cells, increased activity of cytokine generation, increased effect activity of cytotoxic T cells, increased effect activity of macrophages, increased activity of natural killing cells, cancer prevention or treatment activity, automated immune disability prevention or treatment activity, virus infection prevention or treatment activity, prevention or treatment activity of HIV-related diseases, hepatitis C prevention or treatment activity, rheumatoid arthritis prevention or treatment activity, and the like.

The term "expression" used herein refers to the generation of proteins or nucleic acids in cells.

A single letter (three letters) of amino acids used herein means the following amino acids according to standard abbreviation rules in the field of biochemistry: A (Ala): Alanine; C (Cys): Cysteine; D (Asp): Aspartic acid; E (Glu): Glutamic acid; F (Phe): phenylalanine; G (Gly): Glycine; H (His): Histidine; I (Ile): Isoleucine; K (Lys): Lysine; L (Leu): Leucine; M (Met): Methionine; N (Asn): Asparagin; O (Ply): Pyrrollysine; P (Pro): Proline; Q (Gln): Glutamine; R (Arg): Arginine; S (Ser): Serine; T (Thr): Threonine; U (Sec): selenocysteine; V (Val): Valine; W (Trp): Tryptophan; Y (Tyr): Tyrosine.

The present invention provides a method for purifying a diglycosylated interferon-beta protein including steps of:

(a) obtaining a culture solution comprising interferon-beta expressed in a host cell;
(b) performing affinity chromatography for the culture solution obtained in step (a);
(c) low pH inactivating the solution obtained in step (b);
(d) performing hydrophobic interaction chromatography for the solution obtained in step (c);
(e) performing anion-exchange chromatography for the solution obtained in step (d); and
(f) performing cation-exchange chromatography for the solution obtained in step (e).

In the purification method (or separation method) of the present invention, the respective steps are as follows.

Step (a): obtaining a culture solution comprising interferon-beta expressed in a host cell;

The interferon-beta (interferon-beta polypeptide or interferon-beta protein) of the present invention may be interferon-beta that an arginine (R27) site at position 27 of wild-type interferon-beta is mutated to threonin (R27T). Preferably, the interferon-beta of the present invention may be a polypeptide consisting of an amino acid sequence represented by SEQ ID NO: 1. The interferon-beta of the present invention should be understood as a polypeptide having interferon-beta activity, in which a sugar chain binds to two sites of amino acids at positions 25 and 80 of SEQ ID NO: 1. Preferably, the interferon-beta of the present invention represents a human interferon-beta.

The interferon-beta of the present invention may be expressed from a host cell transformed to be expressed so that the gene is expressed to be expressed in an intracellular or extracellular position. In the present invention, the host cell may be selected to regulate the expression of an inserted sequence or proceed with a gene product in a particular manner. Different host cells have characteristic and specific mechanisms for translation of the protein and processing and variation after translation. A suitable cell line or host system may be selected to provide preferable variation and processing of expressed heterogeneous proteins. The expression in yeast may produce biological active products. The expression in eukaryotic cells may increase the likelihood of natural folding.

In the present invention, host cells that may be continuously expressed while stabilizing the interferon-beta may use even any host cells known in the art, but host cells of prokaryotes may not be used to express diglycosylated interferon-beta. The host cells of the present invention may be, for example, yeast (Saccharomyces cerevisiae), insect cells, animal cells and human cells, preferably chinese hamster ovary (CHO), W138, BHK, COS-7, 293, HepG2, 3T3, RIN, and MDCK cell lines, most preferably a CHO cell line.

In the present invention, the culture may be performed under an appropriate condition capable of expressing a target protein, interferon-beta and such a condition may be performed according to a known method. Transformed host cells may be cultured in large quantities by conventional culture methods. The culture medium may be used with a medium consisting of a carbon source, a nitrogen source, vitamins, and minerals, for example, used with a medium such as SFM4CHO, optiCHO SFM, and 200 mM L-glutamine and the like may be used as an additive. The culture of the host cells can be cultured on a conventional host cell culture condition, for example, may be cultured in a temperature range of 15°C to 45°C for 10 hours to 40 hours. In order to remove the culture medium in the culture solution and recover only the concentrated cells, centrifugation or filtration may be performed, and this step may be performed as needed by those skilled in the art.

Step (b): performing affinity chromatography for the culture solution obtained in step (a)

The term "affinity chromatography" used herein means a chromatography method using an affinity chromatography material. In the affinity chromatography, the polypeptides are separated based on its biological activity or chemical structure according to electrostatic interaction, hydrophobic binding and/or hydrogen binding to a chromatographic functional group. In order to recover specifically-bound polypeptides from the affinity chromatography material, competitor ligands are added, or chromatographic conditions such as pH values, polarity or ionic strength of a buffer are changed. The affinity chromatographic material is a chromatography material including a composite chromatography functional group combined with different single chromatographic functional groups so as to bind only to a certain type of polypeptide. This chromatographic material specifically binds to some types of polypeptides according to the specificity of the chromatographic functional group.

In the present invention, the affinity chromatography material may be dye affinity chromatography having affinity with interferon, preferably interferon-beta, and may use Blue Sepharose as a resin. The dye affinity chromatography may be a matrix fixed with, for example, Blue Dextran Sepharose R or Cibacron R Blue. For example, Matrex Gel Blue A of Amicon, Fraktogel 45 TSK AF-Blue of Merck, or Blue-Sepharose R 6FF (fastflow) of GE Healthcare may be used.

In the exemplary embodiment of the present invention, the diglycosylated interferon-beta protein confirmed that an interferon-beta protein is separated, particularly, a glycosylated state is maintained by affinity chromatography using Blue-Sepharose R 6FF and the interferon-beta protein is separated. Accordingly, in the present invention, preferably, Blue-Sepharose R 6FF may be used as the affinity chromatography material.

The separation and purification of interferon-beta by dye affinity chromatography using Blue-Sepharose R 6FF are known in various technical literature in the art, and the method of performing the chromatography in the technical literatures is included in this specification for reference.

The interferon-beta strongly binds to Blue-Sepharose R 6FF, and the bound proteins may be washed with various buffers before elution. Since the interferon-beta binds well to Blue-Sepharose R 6FF even at a low concentration under conventional conditions, this step is suitable as a first chromatography step of the purification process according to the present invention. As a reagent used for elution of IFN-beta, a Chaotropic agent, a salt, or the like may be used, and an acqueous solution including a reducing agent such as ethylene glycol or propylene glycol may be used. Preferably, for elution in this step, a propylene glycol aqueous solution may be used, and 20% to 40% of a propylene glycol aqueous solution may be used.

Step (c): low pH inactivating the solution obtained in step (b)

The term "low pH inactivating" used herein represents inactivating viral external substances under an acidic condition of pH 4.0 or less by adding acid for the purpose of inactivation of enveloped virus.

Preferably, the low pH inactivating is performed to become pH 2.0 to pH 4.0, more preferably pH 3.0 to pH 4.0 or pH 3.5 to pH 4.0 by adding the acid. The treating time and temperature may be 10°C to 30°C or 15°C to 25°C and 30 minutes to 4 hours. After treating, the acidic solution is neutralized to pH 6 to pH 7.5, preferably pH 6.5 to pH 7.5 using a basic solution if necessary, and the subsequent step is performed.

The acid to be added may be inorganic acid, such as phosphoric acid, hydrochloric acid and sulfuric acid, or organic acid, such as acetic acid, and oxalic acid, and preferably a phosphoric acid aqueous solution.

In the embodiment of the present invention, the low pH inactivating has been performed by adding a 10x diluted phosphoric acid aqueous solution of pH 3.5 to the eluate of step (b) to lower the pH to 4 or less and treating the phosphoric acid aqueous solution at 15°C to 25°C for 1 hour, and then the neutralizing was performed using a 2 M Trizma base.

Step (d): performing hydrophobic interaction chromatography for the solution obtained in step (c)

The term "hydrophobic interaction chromatography" used herein means a chromatography method using a hydrophobic interaction chromatography material. The hydrophobic interaction chromatography material is a chromatographic material to which a hydrophobic group, for example, a butyl-, octyl- or phenyl group as a chromatography functional group is bound. The polypeptide may be separated by interacting with a hydrophobic group of the hydrophobic interaction chromatography material by a hydrophobic region exposed to the surface thereof. The interaction between the polypeptide and the chromatographic material may be affected by a temperature, a solvent, and ionic strength of the solvent. Since the movement of amino acid side chains increases and hydrophobic amino acid side chains buried in the polypeptide are accessible at a lower temperature, the increased temperature further increases the interaction between, for example, the polypeptide and the hydrophobic interaction chromatography material. Also, the hydrophobic interaction is also promoted by a kosmotropic salt and is reduced by the kosmotropic salt.

The hydrophobic interaction chromatography material may be, for example, phenyl sepharose CL-4B, 6FF, HP, phenyl superose, octyl sepharose CL-4B, 4FF and butyl sepharose 4FF, using butyl sepharose, phenyl sepharose, and octyl sepharose, preferably butyl sepharose, more preferably butyl sepharose 4FF, as a resin.

Step (e): performing anion-exchange chromatography for the solution obtained in step (d)

The term "anion-exchange chromatography" used herein means a chromatography method using an anion-exchange chromatography material. The anion-exchange chromatography material means a solid-phase polymeric material having an anion-charged group as a chromatography functional group and an anion-exchanged material. Usually, counter ions bind to the anion-exchange chromatography material, and the anion-exchange chromatography material may exchange anions that are similarly charged in the ambient solution with counter ions.

For example, the anion-exchange chromatography may be, for example, chromatography using a commercial anion-exchange chromatography material, and may use anion-exchange chromatography materials provided by manufacturers such as Biorad Laboratories, Cyphergen, GE Healthcare, and the like.

The anion-exchange chromatography may be performed using quaternary ammonium as an anion-exchange resin, preferably a Q resin as a resin, more preferably Capto Q ImpRes.

Preferably, the anion-exchange chromatography is performed first in ion exchange chromatography. This is a step required for effectively removing a basic variant, virus, host cell protein (HCP), host cell DNA (HCD), and endotoxin, which correspond to impurities in the interferon-beta purification process.

Step (f): performing cation-exchange chromatography for the solution obtained in step (e)

The term "cation-exchange chromatography" used herein means a chromatography method using a cation-exchange chromatography material. The cation-exchange chromatography material means a solid-phase polymeric material having a cation-charged group as a chromatography functional group and a cation-exchanged material. Usually, counter ions bind to the cation-exchange chromatography material, and the cation-exchange chromatography material may exchange cations that are similarly charged in the ambient solution with counter ions.

Depending on the properties of the charged group, the cation-exchange chromatography material may have, for example, a sulfonic acid group (SP) or a carboxymethyl group (CM). Further, depending on the properties of the charged group, the cation-exchange chromatography material may be further classified as a strong or weak cation-exchange chromatography material depending on the intensity of a charged substituent. For example, the strong cation-exchange chromatography material has a sulfonic acid group as a chromatography functional group, and the weak cation-exchange chromatographic material has a carboxylic acid group as a chromatography functional group.

For example, the cation-exchange chromatography may be chromatography using, for example, a commercial cation-exchange chromatography material and may use a cation-exchange chromatography material provided by manufacturers of Biorad Laboratories, Cyphergen, GE Healthcare, and the like. The cation-exchange chromatography may be performed by using preferably SP sepharose, more preferably sephadex S.F.F. as a resin.

The interferon-beta separated through the purification of the present invention has the activity of human interferon-beta, has biological and/or immunological characteristics of the interferon-beta to be applicable in general treatment in terms of good manufacturing practices (GMP), and has preferably approval requirements (validation, reproducibility) by regulations and interferon-beta specific biochemical characteristics (such as hydrophobicity, etc.).

The inactivity of the separated diglycosylated interferon-beta may be at least $2 \times 10^8$ IU/mg, preferably at least $3 \times 10^8$ IU/mg, more preferably at least $4 \times 10^8$ IU/mg. Two essential biological activities of the IFN-beta that can be measured are an antiviral effect and an antiproliferative effect. These biological activities may be measured by a standard method through inhibition of a cell modification effect of virus, respectively. The measurement the activity of interferon-beta may use methods known in the art.

The purification method of the diglycosylated interferon-beta of the present invention does not include, preferably reverse-phase chromatography, ultrafiltration or dialysis, and size exclusion chromatography. Since acetonitrile as a generally used buffer is an organic solvent, the reverse-phase chromatography has a disadvantage that additional equipment such as explosion proof equipment is required for mass culture and purification and the ultrafiltration or dialysis has a disadvantage that it is not suitable for mass culture and purification and a large amount of waste liquid may occur. Further, the size exclusion chromatography has a disadvantage that it is not suitable for mass culture and purification.

Preferably, the reason why the reverse-phase chromatography, the ultrafiltration or dialysis, and the size exclusion chromatography are excluded is that it is possible to fundamentally exclude several constraints that affect safety and environments, such as requiring significant labor force, necessarily using expensive equipment requiring many investments, or handling of a flammable solvent, explosion protection, and a waste liquid.

Further, the present invention provides a diglycosylated interferon-beta protein obtained by the purification method of the present invention. Preferably, the interferon-beta protein of the present invention has a base sequence of SEQ ID NO: 1 and has sugar chains in amino acids at positions 25 and 80.

The sugar chains of the interferon-beta protein may be sugar chains having structures of A2G2, FA2G2, FA2G2S1, FA2G2S2, FA2G2S*2, FA3G3S1, FA3G3S3, FA3G3S*3, FA4G4S1, FA4G4S3, FA4G4S4, FA4G4S*3, FA4G4S*4, FA4G4Lac1S3, FA4G4Lac1S*3, FA4G4Lac1S4, FA4G4Lac2S3, and FA4G4Lac1S*3 denoted according to an oxford notation method, preferably, have two sugar chain structures of the sugar chains.

(F = core fucosylated;

A2, biantennary with both GlcNAcs as beta 1-2 linked;

A3, triantennary with a GlcNAc linked beta 1-2 to both mannoses and the third GlcNAc linked beta 1-4 to the alpha 1-3 linked mannose;

A3', triantennary with a GlcNAc linked beta 1-2 to both mannoses and the third GlcNAc linked beta 1-6 to the alpha 1-6 linked mannose;

A4, GlcNAcs linked as A3 with additional GlcNAc beta 1-6 linked to alpha 1-6 mannose;

Gx, number (x) of galactose linked beta 1-4 to antenna;

Sx, number (x) of sialic acids linked to galactose; sialic acid is in an alpha 2-3 unless indicated by a * that alpha 2-6 linkages are also present;

Lacx, number (x) of lactosamine (Gal-GlcNAc) extensions.

The additional possible lactosamine structures are listed in italic)

In the exemplary embodiment of the present invention, cells were cultured in a 5 L scale and interferon-beta was expressed and then purified by using the purification method of the diglycosylated interferon-beta of the present invention. The purification of the diglycosylated interferon-beta was performed by performing sequentially affinity chromatography using a Blue sepharose 6 fast flow resin, hydrophobic interaction chromatography using low pH inactivation by acid addition and a butyl sepharose (butyl 4 fast flow) resin, anion-exchange chromatography using a Capto Q ImpRes resin, and cation-exchange chromatography using a SP sepharose fast flow column.

As Comparative Example, the interferon-beta prepared by the same culture expression was purified according to a conventional purification method of a interferon-beta protein by performing sequentially affinity chromatography using a Blue sepharose 6 fast flow column, reverse-phase chromatography (VYDAC C4) using cation-exchange chromatography using a CM sepharose fast flow resin, ultrafiltration and concentration, and size exclusion chromatography (Sephacryl 100HR).

In the exemplary embodiment of the present invention, the interferon-beta separated and purified according to each purification method was evaluated in terms of yield, protein modification, inactivity, content, and total activity. As a result, the method of the present invention was excellent in the total content, yield, and a Met deletion form as compared with the method of Comparative Example, the method of Comparative Example is high in the inactivity as compared with the method of the present invention, and the method of the present invention is significantly excellent even in the total activity considering the total purification amount. Therefore, it could be seen that the diglycosylated interferon-beta may be separated/purified by the method of the present invention to have excellent quality and yield and higher activity.

In the exemplary embodiment of the present invention, impurities of the interferon-beta separated and purified according to each purification method were confirmed in terms of a cell-derived protein (CHO-HCP) content of CHO cells, an endotoxin content, and patterns in IEF. As a result, it could be confirmed that the protein of the CHO cells and endotoxin were suitable for a general reference value, but the content of impurities was less detected by the method of the present invention. In addition, it could be seen that since there is no basic variant, the method of the present invention was a purification method with much higher purity than the method of Comparative Example 1.

Through a series of processes as described above, the purification yield and purity for each step were shown in Table 1 below.

Table 1

| | Start Material | | | Eluted Pool | | | Yield |
|---|---|---|---|---|---|---|---|
| | Conc. (mg/mL) | Volume (mL) | Amount (mg) | Conc. (mg/mL) | Volume (mL) | Amount (mg) | |
| Blue | N/A | 4,500 | N/A | 0.574 | 66 | 37.9 | N/A |
| Low pH & Neutralization | 0.574 | 64 | 36.7 | 0.544 | 68 | 37.0 | 100.7% |
| HIC Loading Sample Preparation | 0.544 | 64 | 34.8 | 0.131 | 261 | 34.1 | 97.8% |
| HIC | 0.131 | 255 | 33.3 | 0.095 | 123 | 11.7 | 35.1% |
| AEX | 0.095 | 115 | 10.9 | 0.407 | 25 | 10.2 | 93.2% |
| CEX | 0.078 | 150 | 11.7 | 0.290 | 30 | 8.7 | 74% |
| Total Yield | N/A | N/A | N/A | N/A | N/A | N/A | 20.1% |

## ADVANTAGEOUS EFFECTS

The purification method of the interferon-beta protein provided in the present invention is significantly excellent in purification yield of the diglycosylated interferon-beta protein, easy in manufacturing management and quality management in mass culture, and may purify the diglycosylated interferon-beta protein with excellent quality suitable for regulations to be used as medicines.

## DESCRIPTION OF DRAWINGS

FIG. 1 schematically illustrates a purification method of the present invention.

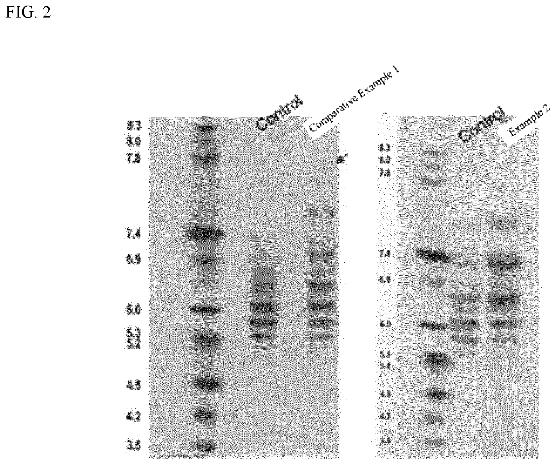

FIG. 2 illustrates a result of performing IEF experiments for materials purified according to a purification method (right panel) of the present invention and a comparative purification method (left panel). A red arrow represents a basic variant.

## MODES FOR THE INVENTION

Hereinafter, the present invention will be described in detail.

However, the following Examples are just illustrative of the present invention, and the contents of the present invention are not limited to the following Examples.

### Example 1: Expression and Obtainment of Diglycosylated interferon-beta in Host Cells

A culture solution expressed interferon-beta by culturing a producing CHO cell line including a corresponding diglycosylated interferon-beta gene in a 5 L scale using a medium based on optiCHO SFM of Hyclone.

The culture solution was cultured while slowly stirring at an initial temperature of 34.0°C, pH 7.0, and 50% dissolved oxygen, and cultured for 9 days, and then the culture solution recovered from an incubator was centrifuged under conditions of 4500 rpm and 4 °C for 30 minutes. After the centrifugation, a supernatant was aseptically subjected to 0.22 μm filtration, and purification was performed.

**Example 2: Purification of Diglycosylated interferon-beta**

The interferon-beta obtained in Example 1 was added to a purification process using affinity chromatography.

First, the harvested interferon-beta fractions were bound with a Blue sepharose 6 fast flow (GE) column pre-equilibrated with a solution of 10 mM sodium phosphate, 137 mMNaCl, 2.7 mM KCl, and pH 7.4, and sufficiently washed with a 700 mM sodium thiocyanate solution. Thereafter, the interferon-beta fractions were eluted with a solution of 20 mM sodium phosphate, 1.4 M NaCl, 35% propylene glycol, 250 mM L-Arginine, and pH 7.4 to be harvested.

As a result of HPLC analysis, purity of 94% to 96% was shown, and as a result of residual HCP detection, the first purification was completed with the content of 6300 to 9500 ppm.

In a second step, pH 3.5 of phosphoric acid (99%, and 10-fold diluted using distilled water) was added to adjust the pH to 3.0. Thereafter, the phosphoric acid was treated at 22°C for 1 hour and then neutralized with 2 M Trizma base to be sampled.

In a third step, the fractions were bound to a butyl sepharose (Butyl 4 fast flow; GE) column pre-equilibrated with a solution of 20 mM Tris, 1.4 M NaCl, 300 mM Arg, and pH 7.4 and then eluted with a solution of 20 mM Tris and pH 8.0 to be harvested.

In a fourth step, anion-exchange chromatography filled with a Capto Q ImpRes (GE) resin using the solution of 20 mM Tris and pH 8.0 was performed using the obtained eluted fractions. The solution for eluting the solvent used 20 mM Tris, 140 mM NaCl, and pH 8.0.

In a final step, the fractions eluted in an anion-exchange resin before were added to a SP sepharose fast flow (GE) column equilibrated with 50 mM sodium acetate and pH 5.0 to perform cation-exchange chromatography by a salt concentration difference from 150 mM to 500 mM.

As a result, finally, the interferon-beta material obtained with yield of 32% exhibited purity of 95% or more, the HCP recorded 100 ppm which was a reference value or less, and as an IEF and SDS-PAGE gel analysis result, a final product with nothing significant was obtained.

**Comparative Example 1: Purification according to conventional purification method for interferon-beta protein**

Interferon-beta fractions obtained from the culture were bound with a Blue sepharose 6 fast flow (GE) column pre-equilibrated with a solution of 10 mM sodium phosphate, 137 mM NaCl, 2.7 mM KCl, and pH 7.4, and sufficiently washed with a 700 mM sodium thiocyanate solution. Thereafter, the interferon-beta fractions were eluted with a solution of 20 mM sodium phosphate, 1.4 M NaCl, 35% propylene glycol, and pH 7.0 to be harvested.

The fractions obtained in the above process were diluted 5-fold with a solution of 20 mM sodium phosphate and pH 2.7 and added to cation-exchange chromatography as a second process using a CM sepharose fast flow (GE) resin. The elution solvent used at this time was 50 mM NaPi, 0.145 M NaCl, 10% PG, and pH 7.0.

Thereafter, the eluted interferon-beta sample was eluted by using acetonitrile 30% as an organic solvent and trifluoracetic acid 0.1% as a buffer solution through an HPLC reverse-phase chromatography colum (VYDAC C4; Agilent). At this time, 20 mM sodium phosphate monobasic pH 2.9 was pre-contained in a collecting container of the eluted sample so that the sample was eluted and diluted at the same time, and the interferon-beta was obtained.

In the previous process, a 7-fold diluted interferon-beta eluting sample was concentrated about 7 times through a TFF system (Pellicon Biomax 10; Millipore) of Millipore for ultrafiltration and concentration, in order to be purified by the most efficient method while reducing the capacity. At this time, the exchanging solution was used as 20 mM sodium phosphate pH 2.9, and the concentration of the concentrate was generally based on the resin properties of size exclusion chromatography.

As a final process, unnecessary materials were removed using size exclusion chromatography (Sephacryl 100HR; GE), and at this time, the eluted sample was collected by 25 mL, and finally, the fractions including interferon-beta were obtained.

**Example 3: Evaluation of purification method**

**3-1. Evaluation of activity, yield, etc.**

The purification methods of Example 2 and Comparative Example 1 were evaluated in terms of yield, modification of the protein, inactivity, content, and total activity.

The protein quantification used a UV measurement method. A zero point was adjusted by a solution of adding 0.5 M NaOH and a size exclusion chromatography eluting solvent at 1:1 and a product obtained in the size exclusion chromatography and 0.5 M NaOH were mixed at 1:1 and measured at UV wavelength 290 nm. The measurement was based on three times and was digitized by the following Equation.

$$- \text{(Average of 3 measured values} \div \text{extinction coefficient)} \times \text{dilute magnification}$$

$$\text{(mg/mL)}$$

The yield was based on an elute (100%) passing through blue sepharose and a relative content thereto was measured by an enzyme-linked immunosorbent assay (ELISA) using a primary antibody specific to interferon-beta, and the ELISA used at this time was as follows. After an enzyme-conjugated antibody solution was dispensed in a measurement plate according to a concentration, a standard solution and a test solution to be measured were added two times to at least 8 dilution concentrations. Thereafter, a washing process was subjected after a reaction for a certain time, and then a substrate solution causing a coloring reaction was added to induce the reaction. After the reaction was stopped, the absorbance was measured at 450 nm.

The protein modification was measured by performing peptide mapping using HPLC equipment. An analytical sample was injected using a chromatographic system and a meter, and a protein structure and amino acid denaturation corresponding to a retention value and an integral value were confirmed with respect to values measured at a wavelength of 214 nm.

The inactivity was measured as follows by a cytopathic effect (CPE) assay. A standard material for measuring the inactivity was prepared as follows: Interferon-beta purchased by the National Institute for Biological Standads and Control (NIBSC) was mixed in an MEM culture medium to make an appropriate activity standard stock solution (2,000 IU/mL)) and then prepared by diluting STD activity (IU/mL) sequentially from 200 to 0.390625 IU/mL.

Each purified sample was diluted using an MEM culture medium so that expected activity become 50 IU/mL (volume was 1500 $\mu$l or higher) and diluted sequentially from 50 to 0.390625 IU/mL to prepare a measuring sample.

The measuring sample and the standard material were dispensed in a 96-well culture plate, respectively, and A549 cells were added to wells at $3 \times 10^4$ cells/well and then incubated for 22 hours under 37°C and 5% $CO_2$ conditions. The medium was removed from the plate, and a virus solution (1000 TCID50/ml) was dispensed in each well, and incubated for 22 hours under 37°C and 5% $CO_2$ conditions. The solution was removed from the plate and then stained with a Crystal Violet solution at room temperature, and bleached with 2-Methoxyethanol, and then the absorbance was measured at 570 nm to calculate the inactivity.

The results of respective analysis items were shown in the following table.

Table 2

| Analysis item | Comparative Example 1 | Example 2 |
|---|---|---|
| Final product Total quantity (UV) | 16.5mg | 26.4mg |
| Yield (ELISA, First) | 10.9% | 19% |
| Yield (ELISA, Second) | 11.5% | 20.1% |
| N'-Met deletion form (Peptide mapping) | 3.6% | 2.8% |
| Specific activity (CPE/UV) | 327 MIU/mg | 247 MIU/mg |
| Total activity | 5395 MIU | 6520 MIU |

As the measuring result, in the method of Comparative Example 1, the total content was 16.5 mg, the yield was about 11%, and a Met deletion form was shown as 3.6%. The inactivity was higher than the method of the present invention as 327 MIU/mg, and the purified content was not only about 16.5 mg, and the total activity was only 5395 MIU.

On the contrary, in the method of the present invention, the total content was 26.4 mg, the yield was about 20%, the Met deletion form was 2.8%, and the total activity was high as 6520 MIU.

Therefore, it could be seen that the diglycosylated interferon-beta may be separated/purified by the method of the present invention to have excellent quality and yield and higher activity.

**3-2. Evaluation of impurities**

In the purification of Comparative Example 1 and Example 2, the impurities were confirmed in terms of a cell-derived protein (CHO-HCP) content of CHO cells, an endotoxin content, and patterns in IEF.

A CHO cell-derived protein content to be separated from the interferon-beta as the target protein was measured by using the ELISA. A dilution solution was prepared and dispensed in the plate and the analytical sample was diluted to the final half. In addition to the diluted sample, the standard solution was transferred and dispensed to a plate included in an analytical kit (wherein the enzyme-conjugated antibody was applied to a kit-attached plate), and a substrate solution representing the coloring reaction was added to induce the reaction. Thereafter, in the plate, the CHO cell-derived protein amount to a standard calibration curve was analyzed by measuring the absorbance at a wavelength of 450 nm.

Limulus amebocyte lysate (LAL) assay was used to measure a remaining endotoxin level. An endotoxin standard sample was mixed well with LAL analysis-dedicated distilled water, and then prepared at a concentration to draw the standard calibration curve in a test tube for analysis. A standard sample, a zero point solution, and an analysis sample for each concentration prepared were dispensed to the plate at the same amount. An LAL reagent was well dissolved in the analysis-dedicated distilled water, and then uniformly dispensed to the above prepared plate to induce the reaction. In the plate after the reaction ended, an endotoxin level present in a target material was measured by measuring the absorbance at a wavelength of 405 nm.

Positive and negative buffers to be used for IEF gel analysis were prepared and mixed with an IEF analysis buffer (pH 3 to 10) by calculating the amount of the analystic sample and the standard sample to be the same as each other to make a development sample for analysis. An IEG gel of pH 3 to 10 was attached to a development container and then the positive buffer was filled in the development container and the negative buffer was filled in an external space, and the prepared samples were injected to the gel. The samples were developed in the prepared development container in the order of 200 V for 1 hour and 500 V for 30 minutes and then the completed gel was separated and subjected to coomassie dyeing and bleaching, and isoelectric points of the protein was determined through a chemiluminescence analyzer.

Table 3

| Analysis item | Comparative Example 1 | Example 2 |
|---|---|---|
| CHO-HCP | 65ppm | 42ppm |
| Endotoxin | 0.00 EU/ug | 0.00 EU/ug |
| IEF Result (Fig 1) | Confirm basic variant | Non-confirm basic variant |

As a result, the protein content of CHO cells was 65 ppm in Comparative Example 1 and 42 ppm according to the method of the present invention. Although the protein contents of Comparative Example 1 and the present invention were presented to 100 ppm or less as a general reference value, in the present invention, it was confirmed that the protein content was slightly low by performing cation-exchange chromatography following anion-exchange chromatography.

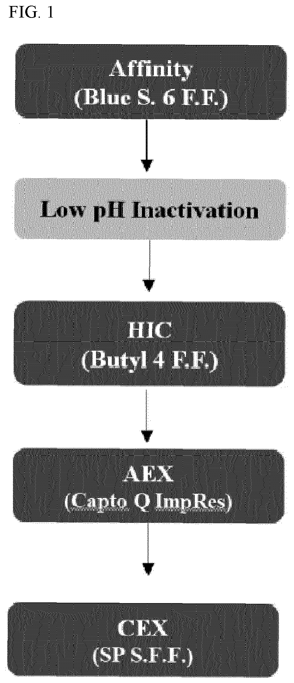

The endotoxin was not contained in both cases, and as a result of confirming the IEF result (FIG. 1), it was confirmed that in Comparative Example 1, a very small amount of basic variant was confirmed around about pH 7.7 (arrow), while in the method of the present invention, the basic variant was not confirmed and the method was a purification method with higher purity.

## INDUSTRIAL APPLICABILITY

As described above, the purification method of the interferon-beta protein provided in the present invention is significantly excellent in purification yield of the diglycosylated interferon-beta protein, easy in manufacturing management and quality management in mass culture, and may purify the diglycosylated interferon-beta protein with excellent quality suitable for regulations to be used as medicines, and thus has large industrial applicability.

## Claims

**1.** A method for purifying a diglycosylated interferon-beta protein comprising the following steps:

(a) obtaining a culture solution comprising interferon-beta expressed in a host cell;
(b) performing affinity chromatography for the culture solution obtained in step (a);
(c) low pH inactivating the solution obtained in step (b);
(d) performing hydrophobic interaction chromatography for the solution obtained in step (c);
(e) performing anion-exchange chromatography for the solution obtained in step (d); and

(f) performing cation-exchange chromatography for the solution obtained in step (e).

2. The method of claim 1, wherein the interferon-beta protein consists of a peptide sequence of SEQ ID NO: 1.

3. The method of claim 1, wherein the host cell is a chinese hamster ovary (CHO) cell.

4. The method of claim 1, wherein the affinity chromatography is performed using a blue sepharose as a resin.

5. The method of claim 1, wherein the hydrophobic interaction chromatography is performed using a butyl sepharose as a resin.

6. The method of claim 1, wherein the anion-exchange chromatography is performed using a Q resin as a resin.

7. The method of claim 1, wherein the cation-exchange chromatography is performed using SP sepharose as a resin.

8. A diglycosylated interferon-beta protein obtained by the purification method of claim 1.

9. The diglycosylated interferon-beta protein of claim 8, wherein the interferon-beta protein has a base sequence of SEQ ID NO: 1 and has sugar chains at amino acid positions 25 and 80.

10. The diglycosylated interferon-beta protein of claim 8, wherein the interferon-beta protein includes any two sugar chain structures of A2G2, FA2G2, FA2G2S1, FA2G2S2, FA2G2S*2, FA3G3S1, FA3G3S3, FA3G3S*3, FA4G4S1, FA4G4S3, FA4G4S4, FA4G4S*3, FA4G4S*4, FA4G4Lac1S3, FA4G4Lac1S*3, FA4G4Lac1S4, FA4G4Lac2S3, and FA4G4Lac1S*3.

FIG. 1

FIG. 2

## INTERNATIONAL SEARCH REPORT

| International application No. |
|---|
| **PCT/KR2020/008847** |

### A. CLASSIFICATION OF SUBJECT MATTER

**C07K 1/18**(2006.01)i; **C07K 1/20**(2006.01)i; **C07K 1/22**(2006.01)i; **C07K 14/565**(2006.01)i

According to International Patent Classification (IPC) or to both national classification and IPC

### B. FIELDS SEARCHED

Minimum documentation searched (classification system followed by classification symbols)

C07K 1/18; A61K 38/21; A61K 47/18; C07K 14/565; C07K 16/28; C12P 21/02; C07K 1/20; C07K 1/22

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 2당화된 인터페론-베타(double-glycosylated interferon-beta), 정제(purification), 친화성 크로마토그래피(affinity chromatography), 산성 불활성화(acidic inactivation), 소수성 상호작용 크로마토그래피 (hydrophobic interaction chromatography), 음이온 교환 크로마토그래피(anion exchange chromatography), 양이온 교환 크로마토그래피(cation exchange chromatography)

### C. DOCUMENTS CONSIDERED TO BE RELEVANT

| Category* | Citation of document, with indication, where appropriate, of the relevant passages | Relevant to claim No. |
|---|---|---|
| Y | SONG, Kyoung et al. Glycoengineering of Interferon-β 1a Improves Its Biophysical and Pharmacokinetic Properties. PLOS ONE. 2014, vol. 9, no. 5, Article No. e96967, pp. 1-14, Supporting information. See abstract; pages 2-3; and figure S1. | 1-10 |
| Y | KR 10-2000-0069664 A (BIOGEN, INC.) 25 November 2000. See pages 4-5. | 1-10 |
| A | 손다진 등. 다발성경화증 치료를 위한 인터페론 베타의 산업적 생산. (SON, Da-Jin et al. Industrial Production of Interferon Beta for the Treatment of Multiple Sclerosis.). Korean Society for Biotechnology and Bioengineering Journal. 2018, vol. 33, no. 2, pp. 70-75. See pages 72 and 74; and figure 1. | 1-10 |
| A | JP 2012-532167 A (BIOGENERIX AG) 13 December 2012. See abstract; and claims 1-5 and 13. | 1-10 |
| A | US 2015-0203583 A1 (ABBVIE BIOTHERAPEUTICS INC.) 23 July 2015. See paragraph [0031]; and claims 17-18. | 1-10 |

✓ Further documents are listed in the continuation of Box C.      ✓ See patent family annex.

| | |
|---|---|
| * Special categories of cited documents:<br>"A" document defining the general state of the art which is not considered to be of particular relevance<br>"D" document cited by the applicant in the international application<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **13 October 2020** | **13 October 2020** |

| Name and mailing address of the ISA/KR | Authorized officer |
|---|---|
| **Korean Intellectual Property Office**<br>**Government Complex Daejeon Building 4, 189, Cheongsa-ro, Seo-gu, Daejeon, Republic of Korea**<br>**35208** | |
| Facsimile No. **+82-42-481-8578** | Telephone No. |

Form PCT/ISA/210 (second sheet) (July 2019)

**INTERNATIONAL SEARCH REPORT**

| International application No. |
| --- |
| **PCT/KR2020/008847** |

**C.** **DOCUMENTS CONSIDERED TO BE RELEVANT**

| Category* | Citation of document, with indication, where appropriate, of the relevant passages | Relevant to claim No. |
| --- | --- | --- |
| A | KR 10-2008-0048490 A (ARES TRADING S.A.) 02 June 2008. See claims 1 and 6. | 1-10 |

Form PCT/ISA/210 (second sheet) (July 2019)

| INTERNATIONAL SEARCH REPORT<br>Information on patent family members | International application No.<br>**PCT/KR2020/008847** |
|---|---|

| Patent document<br>cited in search report | | | Publication date<br>(day/month/year) | Patent family member(s) | | | Publication date<br>(day/month/year) |
|---|---|---|---|---|---|---|---|
| KR | 10-2000-0069664 | A | 25 November 2000 | AT | 270899 | T | 15 July 2004 |
| | | | | AU | 5619198 | A | 17 July 1998 |
| | | | | BG | 103594 | A | 28 April 2000 |
| | | | | BG | 65418 | B1 | 31 July 2008 |
| | | | | CN | 1245434 | A | 23 February 2000 |
| | | | | CN | 1733296 | B | 26 May 2010 |
| | | | | EP | 0948358 | A1 | 13 October 1999 |
| | | | | EP | 0948358 | B1 | 14 July 2004 |
| | | | | EP | 0948358 | B2 | 23 November 2011 |
| | | | | HK | 1025040 | A1 | 08 April 2005 |
| | | | | HK | 1092048 | A1 | 15 October 2010 |
| | | | | JP | 2001-519770 | A | 23 October 2001 |
| | | | | JP | 2012-006979 | A | 12 January 2012 |
| | | | | JP | 2014-098029 | A | 29 May 2014 |
| | | | | JP | 4878664 | B2 | 15 February 2012 |
| | | | | JP | 5851695 | B2 | 03 February 2016 |
| | | | | KR | 10-1042660 | B1 | 20 June 2011 |
| | | | | KR | 10-2007-0052363 | A | 21 May 2007 |
| | | | | NZ | 336548 | A | 28 September 2001 |
| | | | | NZ | 512792 | A | 26 November 2002 |
| | | | | US | 2003-0190307 | A1 | 09 October 2003 |
| | | | | US | 2013-0302281 | A1 | 14 November 2013 |
| | | | | US | 2015-0093359 | A1 | 02 April 2015 |
| | | | | US | 8932574 | B2 | 13 January 2015 |
| | | | | US | 9522174 | B2 | 20 December 2016 |
| | | | | WO | 98-28007 | A1 | 02 July 1998 |
| JP | 2012-532167 | A | 13 December 2012 | DE | 102009032179 | A1 | 13 January 2011 |
| | | | | EA | 201290040 | A1 | 30 July 2012 |
| | | | | EP | 2451470 | A1 | 16 May 2012 |
| | | | | US | 2012-0177603 | A1 | 12 July 2012 |
| | | | | WO | 2011-003600 | A1 | 13 January 2011 |
| | | | | WO | 2011-003600 | A8 | 01 September 2011 |
| US | 2015-0203583 | A1 | 23 July 2015 | AU | 2012-203095 | A1 | 13 December 2012 |
| | | | | AU | 2012-203095 | B2 | 04 February 2016 |
| | | | | BR | 102012012673 | A2 | 17 November 2015 |
| | | | | CA | 2777978 | A1 | 27 November 2012 |
| | | | | CN | 102796705 | A | 28 November 2012 |
| | | | | CN | 107090040 | A | 25 August 2017 |
| | | | | EP | 2527429 | A2 | 28 November 2012 |
| | | | | EP | 2527429 | A3 | 30 October 2013 |
| | | | | JP | 2012-244993 | A | 13 December 2012 |
| | | | | JP | 2018-134091 | A | 30 August 2018 |
| | | | | JP | 2020-078348 | A | 28 May 2020 |
| | | | | JP | 6055615 | B2 | 27 December 2016 |
| | | | | MX | 2012006115 | A | 04 December 2012 |
| | | | | MX | 339533 | B | 30 May 2016 |
| | | | | US | 2015-0197573 | A1 | 16 July 2015 |
| | | | | US | 2020-0157230 | A1 | 21 May 2020 |
| | | | | US | 9260528 | B2 | 16 February 2016 |
| | | | | US | 9815903 | B2 | 14 November 2017 |

Form PCT/ISA/210 (patent family annex) (July 2019)

| INTERNATIONAL SEARCH REPORT | International application No. |
| Information on patent family members | **PCT/KR2020/008847** |

| Patent document cited in search report | | | Publication date (day/month/year) | Patent family member(s) | | | Publication date (day/month/year) |
|---|---|---|---|---|---|---|---|
| KR | 10-2008-0048490 | A | 02 June 2008 | AU | 2005-335900 | A1 | 01 March 2007 |
| | | | | AU | 2005-335900 | B2 | 29 March 2012 |
| | | | | CN | 101282990 | A | 08 October 2008 |
| | | | | CN | 101282990 | B | 03 April 2013 |
| | | | | DK | 1917276 | T3 | 07 May 2018 |
| | | | | EA | 015901 | B1 | 30 December 2011 |
| | | | | EP | 2390263 | A1 | 30 November 2011 |
| | | | | ES | 2664924 | T3 | 24 April 2018 |
| | | | | HK | 1120052 | A1 | 13 December 2013 |
| | | | | JP | 2009-505645 | A | 12 February 2009 |
| | | | | JP | 4944112 | B2 | 30 May 2012 |
| | | | | KR | 10-1276367 | B1 | 25 June 2013 |
| | | | | MX | 2008002596 | A | 14 March 2008 |
| | | | | SG | 155183 | A1 | 30 September 2009 |
| | | | | US | 2008-0219952 | A1 | 11 September 2008 |
| | | | | US | 2011-0305669 | A1 | 15 December 2011 |
| | | | | US | 8993724 | B2 | 31 March 2015 |
| | | | | WO | 2007-022799 | A1 | 01 March 2007 |

Form PCT/ISA/210 (patent family annex) (July 2019)

**EP 4 001 291 A1**

**Patent documents cited in the description**

- KR 1020190087244
- US 20120177603 A